# EUROPEAN PATENT APPLICATION

(11) **EP 4 434 561 A1**
(43) Date of publication of application: **25.09.2024**
(21) Application number: 23162908.0
(22) Date of filing: 20.03.2023
(51) Int. Cl.: A61M 5/32, A61M 5/42, A61M 5/46, A61M 39/02, A61M 39/04, A61M 5/158

(54) **PORT CANNULA SYSTEM WITH A FLEXIBLE TUBE**

(71) Applicant: Fresenius Kabi Deutschland GmbH, 61352 Bad Homburg (DE)
(72) Inventor: DIMNI, Oltjon, 36251 Bad Hersfeld (DE); PÜTTER, Harry, 36364 Bad Salzschlirf (DE)
(74) Representative: Fresenius Kabi Deutschland GmbH

(57) **Abstract**

A port cannula system (1) for puncturing a port (2) comprises: a cannula (10) and a catheter (11; 11') having a flexible tube (110) surrounding the cannula (10). The port cannula system (1) further comprises a fixation element (12; 12') adapted to fix the catheter (11; 11) to the skin (3) of a person such that the fixation element (12; 12') is arranged between the catheter (11; 11') and the skin (3).

## Description

The invention relates to a port cannula system for puncturing a port.

Port cannulas are used for puncturing implanted port catheters. An implanted port catheter, in short also referred to as port, is arranged under the skin of a patient and typically defines a volume covered by a membrane. The volume of the port is connected to a catheter that opens into a vessel or other treatment site. To administer a liquid into the port, a port cannula with a needle is used. The needle is pressed through the skin of the patient and through the membrane into the volume so that the liquid can be filled into the volume of the port and, thereby, administered to the patient.

Such a port is described in US 2013/0226103 A1.

Depending on weight and size of the patient, the thickness of the tissue covering the implanted port may differ strongly. Therefore, commonly a cannula with a correct needle length must be selected before the application. The needle length is selected to be just long enough to reach the volume of the port and at the same time allow a connecting piece of the port cannula to be in contact with the patient's skin to be secured thereto.

Due to the wide range of weights and sizes of the various patients, hospitals typically have to store a significant number of different needle lengths, e.g., six different lengths. Since regularly also different needle diameters may be applied, e.g., three different diameters, a large number of different port cannulas may have to be kept in a storeroom. In addition, the manufacturing of the port cannulas is complex, because the various different combinations of needle diameters and lengths need to be made.

US 5,135,502 A proposes a solid introducer for a catheter to a port with the same disadvantages as described above. DE 697 30 513 T2 describes a security-catheter arrangement which is applicable for different weights and sizes of the various patients. However, these two solutions are relatively inconvenient in use and, therefore, both have not been accepted in wider practice.

It is an object of the instant invention to provide an improved port cannula system.

This object is achieved by means of a port cannula system comprising the features of claim 1.

Accordingly, a port cannula system for puncturing a port comprises a cannula and a catheter having a flexible tube surrounding the cannula. The port cannula system further comprises a fixation element. The fixation element is adapted to fix the catheter to the skin of a person such that the fixation element is arranged between the catheter and the skin.

This is based on the finding that known solutions are neither convenient for healthcare personnel in the application nor comfortable for the patient. The fixation element of the proposed solution both provides a reference and an aid for correctly applying the catheter to the port at the required depth, as well as a secure and comfortable fixture of the catheter on the skin. Since the tube is flexible, the catheter may be bent so as to extend in parallel to the skin.

The fixation element may comprise a locking portion for removably locking the catheter to the fixation element. By this, the catheter may be fixed on the skin in a particularly simple and reliable manner.

The fixation element has a skin-facing side for contacting the skin when the fixation element is fixed to the skin. The locking portion may be arranged on an upper side of the fixation element. The upper side is, e.g., opposite the skin-facing side. As such, the port cannula system can be quickly and easily applied in a secure manner.

Optionally, the skin-facing side is provided with an adhesive for fixation to the skin. By this, the application can be particularly reliable and simple.

The locking portion and the catheter may form a snap-in connection. Alternatively or in addition, the locking portion and the catheter may be engageable with one another. Alternatively or in addition, the locking portion and the catheter may comprise a hook-and-loop fastener. These variants allow particularly simple and quick applications.

The port cannula system may comprise a scale configured to indicate a length of the flexible tube pierced into the skin. This can simplify the application and improve the precision thereof.

Optionally, the scale is provided on the fixation element, wherein an indicator indicating the length (of the flexible tube pierced into the skin) on the scale is provided on the catheter. Alternatively, the scale may be provided on the flexible tube, wherein an indicator indicating the length (of the flexible tube pierced into the skin) on the scale is provided on the fixation element. This allows to easily adjust the correct length without additional components. For example, the length may be documented and used for another infusion at a later point in time if the patient has not gained or lost weight.

The catheter may comprise a connector for establishing a fluid connection between the flexible tube and another part, e.g., a connection hose. By this, a simple construction being convenient in use can be provided.

The port cannula system may further comprise a tip protection element removably arranged at the connector and adapted to cover a tip of the cannula when the cannula is withdrawn from the catheter. This allows to prevent needle-stick injuries.

The fixation element may comprise a hole. The catheter (more precisely, the flexible tube thereof) may extend through the hole and/or may be adapted to extend through the hole. This allows a reliable functionality and a protected puncture site. Furthermore, the position of the flexible tube is secured with respect to the puncture site and port via the fixation element. Unintentional removal of the flexible tube, for example upon movement of the patient is prevented. The fixation element comprises for example a plate. In the state fixed to the skin, the plate is in planar abutment with the skin. The plate is, e.g., made of a firm, yet flexible material such as silicone to be comfortable to wear for the patient. A hole is formed in the plate. The flexible tube of the catheter extends through the hole, into the skin and inside the port under the skin.

The flexible tube may comprise a plastics material. By this, a reliable function can be provided. The cannula may be made of a metal, e.g., steel.

Optionally, the flexible tube comprises a reinforcement, e.g., at its distal end. The reinforcement may have a larger stiffness than other portions of the flexible tube. By this, insertion of the flexible tube into the port membrane may be simplified. In addition, the reinforcement can support the flexible tube against elastic forces of the port membrane. This allows to avoid that the membrane squeezes the flexible tube together. In addition, a softer material can be used for the flexible tube.

The reinforcement may comprise, or consist of, a ceramics material, an elastically bendable metal or a plastics material being more rigid than the plastics material of the flexible tube. This allows to further improve the reinforcement.

The distal end of the flexible tube may be tapered, e.g., sharpened. This allows to facilitate the application of the catheter.

The port cannula system may further comprise an implantable port comprising a membrane adapted to be punctured by the cannula and the flexible tube surrounding the cannula.

The idea underlying the invention shall subsequently be described in more detail by referring to the embodiments shown in the figures. Herein:
- Fig. 1: shows an implanted port catheter;
- Fig. 2: shows a catheter and a fixation element of a port cannula system, the catheter being in connection with the implanted port catheter according to Fig. 1;
- Fig. 3A: shows the port catheter system with the catheter and the fixation element according to Fig. 2 as well as a cannula arranged within the catheter;
- Fig. 3B: shows the catheter and the fixation element according to Fig. 3 after removal of the cannula;
- Fig. 3C: shows the catheter and the fixation element according to Fig. 3 after fixing a body of the catheter to the fixation element;
- Fig. 4: shows a cross section of the catheter and the fixation element according to Fig. 3C;
- Fig. 5: shows a port catheter system with a cannula, a catheter and a fixation element;
- Fig. 6: shows a distal end of the catheter according to Figs. 2 and 5; and
- Figs. 7A and 7B: show optional protection elements for the cannula.

Subsequently, port cannula systems for puncturing a port shall be described. The embodiments described herein shall not be construed as limiting for the scope of the invention.

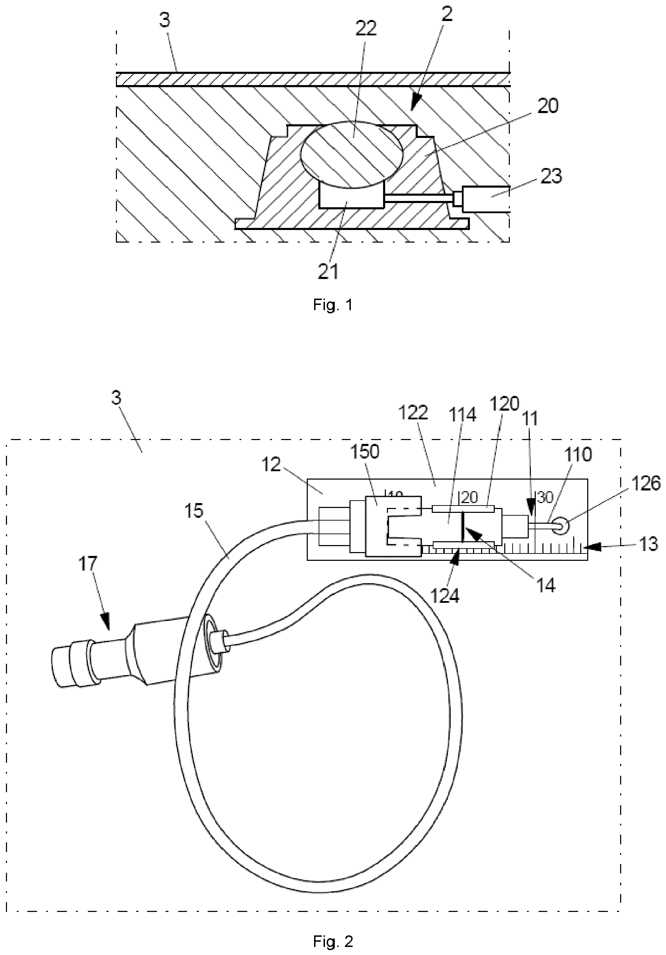

Fig. 1 shows an implantable port catheter, in the following referred to as port 2. In Fig. 1, the port 2 is shown in a state implanted under the skin 3 of a person. The port 2 comprises a housing 20which defines an interior volume 21. A membrane 22 of the port 2 closes the volume 21. The volume 21 may also be referred to as infusion chamber. A catheter 23 is attached to the housing 20 and in fluid connection with the volume 21. The catheter 23 establishes a connection of the port 2 to a tissue, for example a vein. In order to give a patient a medicament that is to be administered intravenously, for example, the membrane 22 is pierced via the skin 3 by means of a port cannula. The port cannula establishes the connection between the infusion chamber volume 21 formed in the housing 20, e.g., to an infusion bag. As an example, a port catheter like the port 2 shown in Fig. 1 can have an implantation time of up to 5 years. Accordingly, the port 2 may be punctured often by a port cannula. The membrane 22 is made of a suitable material, e.g., silicone.

Figs. 2-4 illustrate a port cannula system 1 for puncturing the port 2 of Fig. 1.

The port cannula system 1 comprises a cannula 10, a catheter 11 device with a flexible tube 110 surrounding the cannula 10, and a fixation element 12 adapted to fix the catheter 11 to the skin 3 of a person such that the fixation element 12 is arranged between the catheter 11 and the skin 3.

Fig. 2 shows the fixation element 12 in a state adhered to the skin 3, wherein a body 114 of the catheter 11 is mounted on the fixation element 12. The flexible tube 110 is mounted on the body 114. The fixation element 12 comprises a plate 127 which, in the present example, consists of silicone which allows a comfortable use by its flexibility. In the state fixed to the skin 3 as shown in Fig. 2, the plate 127 is in planar abutment with the skin 3. A hole 126 is formed in the plate 127. The flexible tube 110 of the catheter 11 extends through the hole 126, into the skin 3 and inside the port 2 under the skin 3.

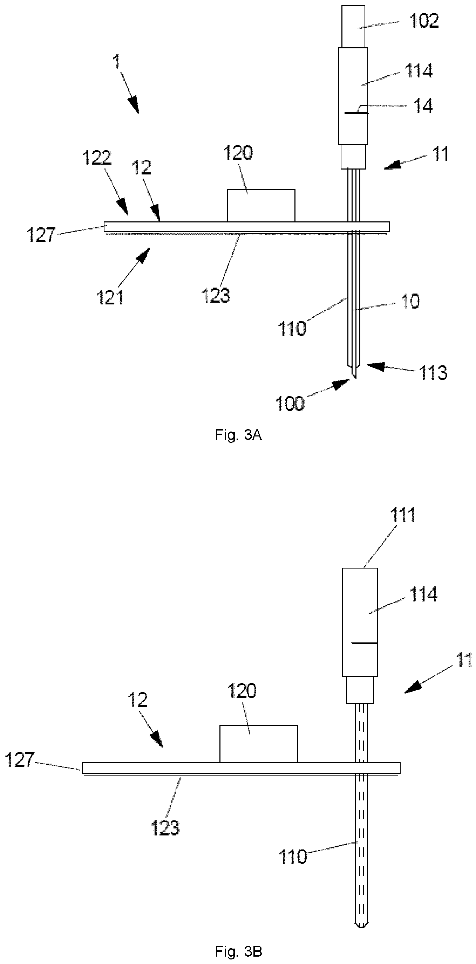

Before turning back to Fig. 2, reference is now made to Figs. 3A-3C.

Fig. 3A shows a side view of the port cannula system 1. The cannula 10 is arranged in the flexible tube 110 of the catheter 11. As such, the rigid cannula 10 stiffens the flexible tube 110. A tip 100 of the cannula 10 projects from the flexible tube 110. The tip 100 is sharpened. A distal end 113 of the catheter 11 is arranged adjacent the cannula 10 tip 100. The distal end 113 of the catheter 11 is tapered. Thus, the catheter 11 with the cannula 10 can be punctured into the skin 3 and into the port 2 under the skin 3. When puncturing the port 2, the fixation element 12 may be laid on the skin 3. Alternatively, it may held upwards for using a scale 13 provided on the fixation element 12, see Fig. 2. The body 114 of the catheter 11 comprises an indicator 14, in this example a line. The indicator 14 indicates the puncturing depth on the scale 13.

Figs. 2-4 show that the fixation element 12 has a skin-facing side 121 for contacting the skin 3 when the fixation element 12 is fixed to the skin 3, and an upper side 122 opposite the skin-facing side 121. The skin-facing side 121 and the upper side 122 extend in parallel to one another. The skin-facing side 121 and the upper side 122 are the two large sides of the plate 127. On the skin-facing side 121, an adhesive 123 is disposed. The adhesive 123 serves to adhere the fixation element 12 to the skin.

Further, the fixation element 12 comprises a locking portion 120 for removably locking the catheter 11 to the fixation element 12. The locking portion 120 is arranged on the upper side 122 of the fixation element 12.

As soon as the catheter 11 has been pierced deep enough into the port 2 under the skin 3, the cannula 10 may be removed. The cannula 10 comprises a body 102, which protrudes from the body 114 of the catheter 11. For removing the cannula 10, the body 102 can be used to manually pull the cannula 10 out of the catheter.

Fig. 3B shows the catheter 11 and the fixation element 12 without the cannula 10. Therefore, after removal of the cannula 10, the flexible tube 110 can be easily bent. Thus, after removing the cannula 10, the flexible tube 110 can be bent so as to fix the body 114 of the catheter 11 on the locking portion 120 of the fixation element 12.

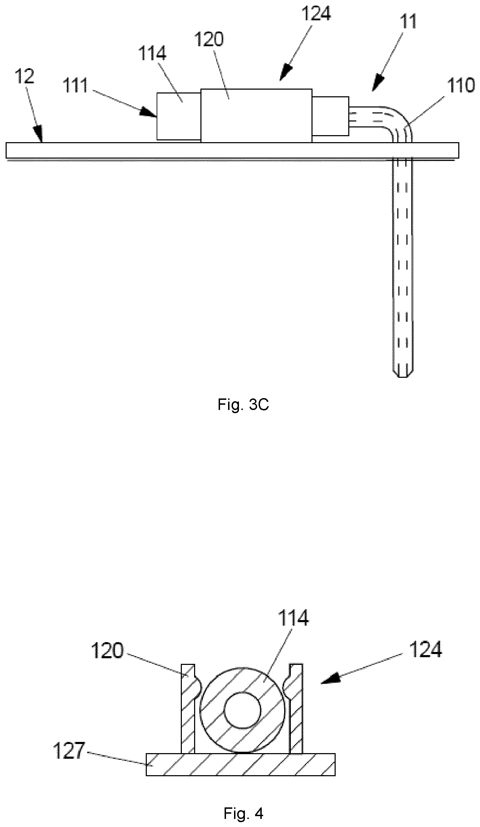

As shown in Fig. 3C, the body 114 then extends substantially perpendicular to the part of the flexible tube punctured into the skin 3 and port 2.

The scale 13 is provided on the upper side 122 of the plate 127 and facilitates the adjustment and inspection of the correct puncturing depth. The scale provides an indication in cm and mm.

In the configuration shown in Figs. 2 and 3C, the point on the skin 3 where the catheter 11 enters the skin 3 is covered by the fixation element 12 the fixation element 12 is rigid. For example, the fixation element 12 is made of plastics. Therefore, the fixation element 12 protects the puncture site. Further, since the body 114 of the catheter 11 is fixedly retained and supported on the fixation element 12, it is laid flat on the skin 3, so that it does not protrude much from the skin 3. This makes it both well protected and securely fixed on the skin 3, as well as comfortable for the patient.

As shown in Fig. 4, the locking portion 120 of this example comprises two sidewalls between which the body 114 of the catheter 11 can be placed. The locking portion 120 forms a snap-in connection 124 together with the body 114. In the present example, the sidewalls of the locking portion 120 comprise inwardly facing protrusions below which the body 114 may be snapped in.

When fixed to the fixation element 12, the body 114 of the catheter 11 extends along the length of the plate 127 of the fixation element 12.

The body 114 of the catheter 11 also forms a connector 111 for connection to a fluid supply. As shown in Fig. 2, a connector 150 of a connection hose 15 can be brought in fluid connection to the connector 111 of the catheter 11. In this example, the connection hose 15 is provided with an NAC or Luer lock 17 for connection with a supply, such as an infusion pump or the like.

The fixation element 12 also facilitates removal of the catheter 11, because the catheter 11 may be pulled while pushing against the fixation element 12.

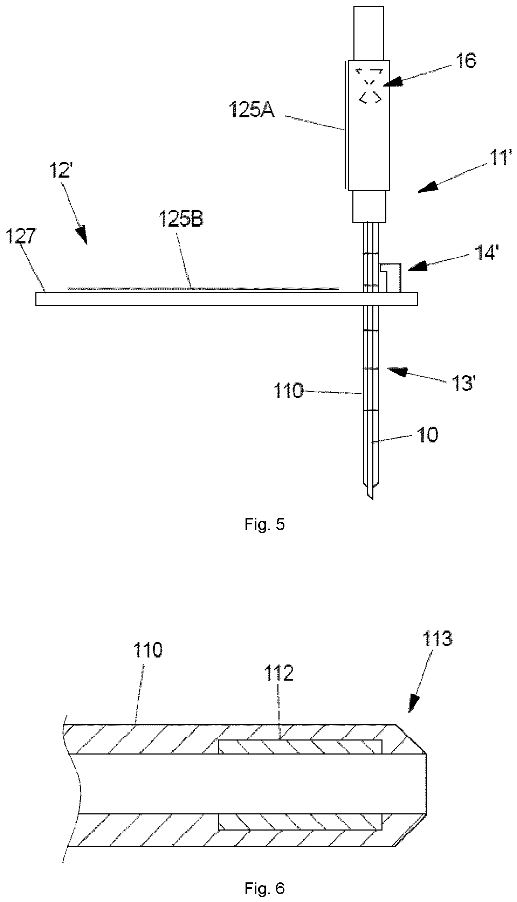

Fig. 5 shows another option for fixing the catheter 11' body to the fixation element 12', namely, using two hook-and-loop fastener 125A, 125B parts. Thus, one part of the hook-and-loop fastener 125A is fixed to the catheter 11' body, the other part of the hook-and-loop fastener 125B is fixed to the fixation element 12', namely to the upper side of the plate 127 thereof.

Further, according to Fig. 5, a scale 13' is provided on the flexible tube 110, wherein an indicator 14' indicating the puncturing length on the scale 13' is provided on the fixation element 12'.

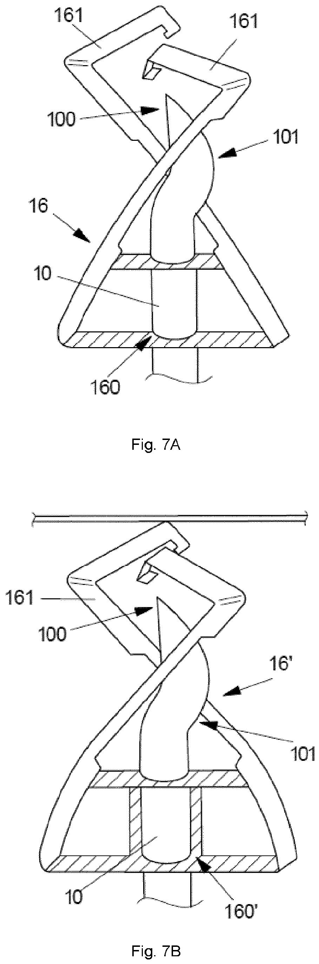

Furthermore, a protection element 16 as described below with reference to Fig. 7A (alternatively in accordance with Fig. 7B) is arranged at (e.g., in) the connector 111 and/or at the body 114 of the catheter 11, and adapted to cover the tip 100 of the cannula 10 when the cannula 10 is withdrawn from the catheter 11.

Fig. 6 shows the distal end 116 of the flexible tube 110 of the catheter 11, 11'. As shown, the flexible tube 110 comprises a reinforcement 112 at its distal end 113. The reinforcement 112 is cylindrical in this example. The reinforcement 112 supports the flexible tube 110 against pressure of the membrane 22 of the port 2 after removal of the cannula 10.

The reinforcement 112 is made of a ceramics material, an elastically bendable metal or a plastics material being more rigid than the plastics material of the flexible tube 110. As an example, the reinforcement 112 may be made of Teflon.

Figs. 7A and 7B show two possible protection elements 16, 16' for the port cannula system 1. Each of the protection elements 16, 16' may be mounted on or in the body 114, e.g., as described above with reference to Fig. 5.

In the present examples, each of the protection elements 16, 16' is made of metal, such as a spring steel. Here, the respective protection elements 16, 16' are made in one piece. Further, the protection elements 16, 16' are made of (flat) sheet material which is bent several times. Each of the protection elements 16, 16' is formed as a needle safety clip. Further, each of the protection elements 16, 16' comprises at least one clamp element 161, here two clamp elements 161, and a needle guide 160, 160'. By means of the needle guide 160, 160', the respective protection element 16, 16' is slidable along the cannula 10. However, the needle guide 160, 160' prevents a form-lock section 101 of the cannula 10 to pass through the needle guide 160, 160'. In this example, a curved portion of the cannula 10 on or adjacent the tip 100 forms the form-lock section 101. The needle guide 160, 160' also prevents a tilting of the protection element 16, 16' with respect to the cannula 10.

When pulling the cannula 10 out of the body 114, the curved portion of the cannula 10 interacts with the needle guide 160, 160' so that the respective protection element 16, 16' is pulled off from the body 114 together with the cannula 10. As such, the respective protection element 16, 16' houses the cannula 10 tip 100. The clamp elements 161 project over the cannula 10 tip 100 and, thereby, can prevent needlestick injuries. The clamp elements 161 cross each other.

The needle guide 160 of the protection element 16 of Fig. 7A comprises two guiding sections that each are in contact with the needle 11 and are spaced apart from one another. Between the two guiding sections the cannula 10 is exposed.

The needle guide 160' of the protection element 16' of Fig. 7B comprises one guiding section forming a cylindrical area of contact with the cannula 10 wherein, in the present example, the cylinder length is larger than the cylinder diameter.

The idea of the invention is not limited to the embodiments described above but may be implemented in a different fashion.

### List of Reference Numerals

- 1: Port cannula system
- 10: Cannula
- 100: Tip
- 101: Form-lock section
- 102: Body
- 11; 11': Catheter
- 110: Flexible tube
- 111: Connector
- 112: Reinforcement
- 113: Distal end
- 114: Body
- 12; 12': Fixation element
- 120: Locking portion
- 121: Skin-facing side
- 122: Upper side
- 123: Adhesive
- 124: Snap-in connection
- 125A, 125B: Hook-and-loop fastener
- 126: Hole
- 127: Plate
- 13; 13': Scale
- 14; 14': Indicator
- 15: Connection hose
- 150: Connector
- 16; 16': Protection element
- 160; 160': Needle guide
- 161: Clamp element
- 17: Luer lock
- 2: Port
- 20: Housing
- 21: Volume
- 22: Membrane
- 23: Catheter
- 3: Skin

## Claims

1. A port cannula system (1) for puncturing a port (2), the port cannula system (1) comprising:
- a cannula (10) and
- a catheter (11; 11') having a flexible tube (110) surrounding the cannula (10),
**characterized by**
- a fixation element (12; 12') adapted to fix the catheter (11; 11') to the skin (3) of a person such that the fixation element (12; 12') is arranged between the catheter (11; 11') and the skin (3).

2. The port cannula system (1) according to claim 1, **characterized in that** the fixation element (12; 12') comprises a locking portion (120) for removably locking the catheter (11; 11') to the fixation element (12; 12').

3. The port cannula system (1) according to claim 2, **characterized in that** the fixation element (12; 12') has a skin-facing side (121) for contacting the skin (3) when the fixation element (12; 12') is fixed to the skin (3), the locking portion (120) being arranged on an upper side (122) of the fixation element (12; 12') opposite the skin-facing side (121).

4. The port cannula system (1) according to claim 3, **characterized in that** the skin-facing side (121) is provided with an adhesive (123) for fixation to the skin (3).

5. The port cannula system (1) according to any of claims 2 to 4, **characterized in that** the locking portion (120) and the catheter (11; 11') form a snap-in connection (124), are engageable with one another and/or comprise a hook-and-loop fastener (125A, 125B).

6. The port cannula system (1) according to any of the preceding claims, **characterized by** a scale (13; 13') configured to indicate a length of the flexible tube (110) pierced into the skin (3).

7. The port cannula system (1) according to claim 6, **characterized in that** the scale (13) is provided on the fixation element (12; 12'), wherein an indicator (14) indicating the length on the scale (13) is provided on the catheter (11) or that the scale (13') is provided on the flexible tube (110), wherein an indicator (14') indicating the length on the scale (13') is provided on the fixation element (12').

8. The port cannula system (1) according to any of the preceding claims, **characterized in that** the catheter (11; 11') comprises a connector (111) for establishing a fluid connection between the flexible tube (110) and a connection hose (15).

9. The port cannula system (1) according to claim 8, **characterized by** a tip protection element (16; 16') arranged at the connector (111) and adapted to cover a tip (100) of the cannula (10) when the cannula (10) is withdrawn from the catheter (11; 11').

10. The port cannula system (1) according to any of the preceding claims, **characterized in that** the fixation element (12; 12') comprises a hole (126), wherein the catheter (11; 11') extends through the hole (126).

11. The port cannula system (1) according to any of the preceding claims, **characterized in that** the flexible tube (110) comprises a plastics material.

12. The port cannula system (1) according to any of the preceding claims, **characterized in that** the flexible tube (110) comprises a reinforcement (112) at its distal end (113).

13. The port cannula system (1) according to claim 12, **characterized in that** the reinforcement (112) comprises, or consists of, a ceramics material, an elastically bendable metal or a plastics material being more rigid than the plastics material of the flexible tube (110).

14. The port cannula system (1) according to any of the preceding claims, **characterized in that** the distal end (113) of the flexible tube (110) is tapered.

15. The port cannula system (1) according to any of the preceding claims, **characterized by** an implantable port (2) comprising a membrane (22) adapted to be punctured by the cannula (10) and the flexible tube (110) surrounding the cannula (10).
